# EUROPEAN PATENT SPECIFICATION

(11) **EP 4 488 811 B1**
(45) Date of publication and mention of the grant of the patent: **06.08.2025**
(21) Application number: 24213753.7
(22) Date of filing: 03.09.2021
(51) Int. Cl.: A61M 16/01, A61M 16/00, A61M 16/10, A61M 16/12, G06F 3/048

(54) **IMPROVEMENTS IN OR RELATING TO PATIENT CARE**
VERBESSERUNGEN IN BEZUG AUF DIE PATIENTENPFLEGE
AMÉLIORATIONS APPORTÉES OU SE RAPPORTANT AUX SOINS DE PATIENT

(30) Priority: 03.09.2020 GB 202013882
(43) Date of publication of application: 08.01.2025
(62) Divisional of application: 21787008.8
(73) Proprietor: Peninsula Medical Technologies Ltd, Totnes, Devon TQ9 5BN (GB)
(72) Inventor: BROWN, Sebastian, Totnes, TQ9 5BN (GB)
(74) Representative: Bryers Intellectual Property Ltd

(56) References cited:
- US-A1- 2012 291 784
- US-B2- 9 199 056
- US-B2- 9 549 687

## Description

The present invention relates generally to anaesthesia and particularly, although not exclusively, to flexible, intelligent and environmentally efficient patient ventilation and/or anaesthesia machines and systems.

### Introduction

The critical care needs of COVID-19 patients were met during the peak of the crisis using anaesthetic machines in intensive care. Hospitals have more anaesthetic machines in operating theatres than ventilators in Intensive Care Units (ICUs) and these can be rapidly re-deployed. Sedation for COVID patients, and almost all ventilated patients use intravenous agents such as Propofol. However, anaesthetic machines can deliver volatile anaesthetics such as Sevoflurane, Isoflurane and Desflurane. Some evidence is emerging that volatile agents offer advantages to IV sedation in ARDS (Acute Respiratory Distress Syndrome) caused by COVID along with other diseases and may reduce mortality. Furthermore, the supply of Propofol became very constrained during the peak of the crisis and hospitals had to use alternative IV sedation methods that are less stable and staff have less familiarity with.

The widespread adoption of the use of volatiles on ICU is limited as there is no waste gas scavenging available and the machines are unfamiliar to ICU nursing staff. Furthermore, the anaesthetic community is very concerned about the environmental impact of volatile anaesthetic agents as they have significant global warming potential (GWP). Desflurane has a GWP20 of 3500, i.e. each kg is equivalent of 3500kg of CO₂. Systems are coming out that can capture and recycle these gases and governmental pressure may mandate the use of these systems. However, anaesthetic machines are not designed to support the efficient capture of anaesthetic agents.

State of the art technologies are focussed around computer algorithm driven volatile anaesthetic use (e.g. GE Aisys CS2). However, while these systems are very efficient during maintenance anaesthesia, significant waste is produced when loading the patient with anaesthetic agent at the start or when waking up, when the anaesthetic agent must be removed. Several issues reduce the effectiveness of recycling systems. Humid waste gases reduce the binding capacity of the absorbent by up to 80%. High pulsatile flows greater than 100L/min during exhalation mean that collection systems are large to prevent back pressure. Scavenging systems pass gas containing no anaesthetic through the absorbent at high flow rates and assemblies are integrated into machines making access difficult. Ventilator drive gases are sometimes mixed with the exhaust gases, increasing flow and diluting the anaesthetic 5-fold. Finally, during cases where no volatiles are used (Total IntraVenous Anaesthesia- TIVA), gas with no volatile anaesthetic strips absorbed material from the collection system.

Anaesthetic machines are needed that are flexible and familiar to staff, designed so that they can facilitate optimal capture of waste gases for recycling for use in the operating theatre and around the hospital. Such machines, with the recycling systems, will enable the anaesthesia service to be used in many locations in the hospitals (ICU, imaging, paediatrics, interventional areas) without expensive scavenging systems. They will enable hospitals to use space more flexibly and adapt to change, reduce the infrastructure costs of theatres and will be there if needed in a pandemic situation to provide effective support with the option of delivering volatiles efficiently with low environmental cost in the ICU.

US 9 199 056 discloses a breathing apparatus connected to a modular respiratory anaesthetic aggregate. US 2012/291784 relates to a medical system for providing ventilator support to a patient and anaesthesia.

Flexible devices that operate across areas influenced by critical care enable a platform for the application of Artificial Intelligence (AI) and Machine Learning (ML) embedded systems that do not require high bandwidth data transfer across the hospital network like cloud-deployed solutions require, but instead store high frequency data on the device, contain hardware to perform the calculations and are configured to do this safely without potentially compromising the performance of a life-sustaining medical device. A flexible device allows an unbroken data record and the transfer of insight between devices so that clinicians can be alerted to deteriorating patients, receive calls for help or view at-risk patients from their own device.

### Description of Technology

The invention is defined by the appended claims. Subject matter referred as embodiments, technologies, aspects and/or disclosures which do not fall under the scope of the claims are not part of the invention.

An aspect of the present technology provides a portable ventilator module including a data module capable of Al and/or ML, the ventilator module is detachably attachable to an anaesthesia module to enable a single device to collect data from a patient in critical care and theatre environments.

An aspect of the present technology provides a system for the unbroken acquisition of data from the entire patient stay in critical care, emergency areas and theatres in a single device by including a data module capable of Al or ML within a portable ventilator module, wherein the portable ventilator module can provide life-sustaining ventilation support to a patient in critical care and wherein the portable ventilator can dock with an anaesthesia module to provide anaesthesia during operations in theatre so that a machine change from an ICU ventilator to a separate anaesthesia machine and back again is not required, thereby avoiding data loss.

Ventilator modules may be provided in combination with an anaesthesia module.

In some aspects and embodiments the present invention provides or relates to a device formed as a modular anaesthetic machine and ventilator.

In some embodiments a ventilator module can be disconnected from an anaesthesia module.

The patient circuit (inspiratory and expiratory tubing) may connect to the ventilator module.

In some embodiments a ventilator module can operate independently of an anaesthesia module.

The ventilator module may, for example, provide one or more of the following:
- Oxygen and air mixing functions to deliver a clinician-set percentage of Oxygen (FiO₂).
- Pressure functions to drive gas into the patient during inspiration and maintain circuit pressure during expiration (PEEP valve).
- Links to a pressurized oxygen supply and the control systems to safely deliver oxygen into the patient circuit
- Sensors and monitoring to control the function of the ventilator and provide information to the user and ensure appropriate alarms
- Power supply with mains connection and battery with smart charging system
- User interface
- Carrying and mechanical supports for portable use

The anaesthesia module may provide additional functionality; for example one or more of the following:
- The ability to administer volatile anaesthetic agents (e.g. Sevoflurane, Desflurane and Isoflurane)
- A pressurized source of nitrous oxide may be provided with the control systems to safely deliver oxygen into the patient circuit
- A pressurized source of medical-grade air may be provided with the control systems to safely deliver oxygen into the patient circuit
- Carbon dioxide absorber
- Adjustable pressure limiting valve and exhaust valve
- Connections to the ventilator module
- Connections that provide a circle system circuit
- Secondary display/user interface

In some aspects and embodiments the technology is modular. An anaesthesia module may be separable from a ventilation module. This means that the machine can stay with the patient through theatre and then into intensive care, with the anaesthesia module being returned to theatre for the next case. This means the same user interface and controls can be used in both environments.

Compressed air is not necessarily required as this may be generated by the same pressurisation system for capturing anaesthetic when the system is not required during anaesthesia mode.

In some embodiments when the anaesthesia module is not connected, air is drawn in from atmosphere through an air mass flow meter by the action of a turbine as in conventional turbine ventilators. Therefore, the turbine serves the function of pressurising air when the anaesthesia module is not present to pressurising the recirculating gas when the anaesthesia module is connected.

In some embodiments, when the anaesthesia module is connected and air is compressed, it can also be passed into an oxygen concentrator and compressor so that oxygen can be stored and generated to enable this anaesthesia machine to operate without oxygen/air or waste gas scavenging.

In some embodiments when the ventilator is used alone, oxygen and air from ambient are blended according to the clinician-set FiO₂ as the gas is drawn into the turbine and pressurised. The gas flow rates therefore change with the flow through the turbine. When the anaesthesia module is connected, the circuit is closed, with the expiratory exhaust connected to the ambient air source, incorporating a CO₂ absorber, reservoir bag and Adjustable-Pressure Limiting Valve (APL). Fresh gas may be added to the closed circle circuit by the oxygen, air and nitrous flow valves at a steady flow rate. This can either be set manually by the clinician or can be determined by the anaesthesia controller to achieve a clinician-set FiO2. The oxygen source can be provided in both ventilation and anaesthesia mode by the ventilator module oxygen supply. Oxygen can also be provided by a separate oxygen supply in the anaesthesia module. This has advantages in the provision of a constant flow of gas for the provision of fresh volatile anaesthetic agent and also to prevent the need for switching the oxygen supply hosing.

The benefits of using a modular system are that the patient connection is not broken when transferring from intensive care (ICU), recovery or emergency areas where the ventilator module is used as a stand-alone ventilator to theatre for surgery. This reduces the risk of harm to the patient from disconnection and reconnection to another device. Furthermore, it maintains the patient data record as only a single device is used across critical care environments and remains with the patient throughout the duration of their critical care episode.

An aspect of the present technology provides a modular anaesthesia machine with a detachable, portable and independent ventilator module containing patient breathing tube connections and a separate, dependent anaesthesia module wherein when separate, the ventilator module is capable of life support ventilation and wherein the ventilator module can dock with the anaesthesia module to provide additional features for the provision of volatile anaesthesia and recirculation of exhaled gases.

An aspect of the present technology provides a modular anaesthesia machine with a detachable, portable and independent ventilator module wherein ambient air is mixed with oxygen for ventilation when the ventilation module is not connected and wherein pressurised air is mixed with pressurised oxygen when the anaesthesia module is connected.

An aspect of the present technology provides a modular anaesthesia machine with a detachable, portable and independent ventilator module in which gas is pressurised for inspiration and to prevent reverse-flow by a turbine.

In some embodiments a turbine is dynamically controlled to adjust circuit flow and pressure.

In some embodiments turbine pressure is variable but patient inspiratory gas flow is provided by the use of an inspiratory valve downstream of the turbine.

In some embodiments a separate anaesthesia module detaches from a ventilator module.

Control of the device, in compliance with the regulatory framework, may be arranged with the ventilator control processor and associated I/O communicating with a Graphical User Interface (GUI) processor to drive the Primary User Interface within the ventilator module. Communication may be provided by CANBUS, although others are envisioned as known to those skilled in the art.

In some embodiments a ventilation module is a master module with a ventilation processor and GUI processor. An anaesthesia module may have its own processor and associated I/O for components within the anaesthesia module.

A smaller display may be linked to the anaesthesia processor and may display the status of the device, whether there is an alarm or fault state and the pressures in the pipeline and cylinder gas supplies along with other parameters (e.g. battery charge level). A larger, secondary display may be provided in the anaesthesia module. However, this may directly connect with the ventilator module when the ventilator module docks with the anaesthesia module.

The anaesthesia module may communicate with the master ventilator module via CANBUS (although other methods can be used).

In some embodiments the ventilation module can be started and operated entirely independently of the anaesthesia module. However, the anaesthesia module may require a ventilation module to complete start-up testing.

The ventilator module may contain components that are required to test the circuit (breathing circuit, turbine etc.) and the large secondary display and the display on the ventilator module are required to perform testing.

In some embodiments, once the anaesthesia module is started with the ventilator module and tests are complete, the ventilator module can be undocked (e.g. at the end of the case) and a different ventilator module docked with the anaesthesia module. This means that the ventilator module can stay with the patient after surgery (going to ICU or to recovery) and another ventilator module can be docked with the anaesthesia module so that the next surgical case can begin.

An aspect of the present technology provides an anaesthesia module controller that interfaces with components in the anaesthesia module and communicates with the ventilator module via CANBUS or other industrial communication method familiar to those skilled in the art via a connection in the docking port that links the anaesthesia and ventilation modules.

The present technology also provides a display for the anaesthesia module, independent of the ventilation module and active when the anaesthesia module is powered on but not necessarily connected to the ventilation module, for parameters specific to the anaesthesia module.

Aspects and embodiments may provide or relate to a system whereby the anaesthesia module can be powered on and tested when connected to a first ventilation module and subsequently disconnected from the first ventilation module and connected to a second ventilation module for the following patient.

Aspects and embodiments may provide or relate to a system whereby the anaesthesia module can be powered on and tested when connected to a first ventilation module and remain connected to the same ventilation module until powered off.

Aspects and embodiments may provide or relate to a system whereby the anaesthesia module can be powered on and tested when connected to a first ventilation module and be disconnected and reconnected to the first ventilation module once or more than once before being powered off.

Aspects and embodiments may provide or relate to a system whereby the ventilation module provides an interface for a secondary display via a docking port that can also be used to connect to an anaesthesia module.

In some embodiments a docking port is provided that links the anaesthesia module to the ventilator module and provides a single point connection for one or more of:
- Recirculated gas input from the anaethesia module to the ventilation module air intake port.
- Exhaled gas output from the ventilation module to the anaesthesia module input
- CANBUS connection between the ventilator processor and the anaesthesia processor.
- Power connection. The anaesthesia module is normally connected to the mains supply as this module is moved less. The power connection allows the ventilator module to operate from this mains supply and to charge the battery in the ventilator module.
- Ethernet connection. The anaesthesia module may be connected to the healthcare facility Local Area Network (LAN). The connection in the docking port allows the connection between the master Ventilator module to the LAN port.
- Pneumatic supply to close the patient circuit valve in case of power failure when the ventilator module is docked to the anaestheia module. This ensures an open circuit when the ventilator module is undocked and powered off (so that a spontaneously breathing patient can breathe unobstructed), but maintains a closed circuit when the ventilator module is docked with the anaestehsia module (where oxygen can be supplied via a manual flowmeter and manual ventilation used via the bag in the anaesthesia module).
- Contacts on both the anaesthesia and ventilator modules to confirm correct docking position.

A docking port may be provided to connect a ventilator module to an anaesthesia module via a single location.

A connection may be provided within the docking port to connect recirculated gas from the anaesthesia module to the air intake input in the ventilator module.

A connection may be provided within the docking port to connect the exhaled gas from the patient from the ventilator module with the recirculation intake of the anaesthesia module.

A communication connection may be provided within the docking port to connect the controller for the ventilator module with the controller for the ventilation module via CANBUS or other industrial or bespoke communication system familiar to those skilled in the art of microcontroller communications.

A connection may be provided within the docking port to pass power from the anaesthesia module to the ventilator module.

An ethernet connection may be provided within the docking port to link the ethernet output from the ventilation module to the healthcare institution Local Area Network.
Contacts may be provided on the ventilator module to confirm correct placement of the ventilator module when docking with the anaesthesia module
Contacts may be provided on the anaesthesia module to confirm correct placement of the ventilator module when docking with the anaesthesia module
Contacts may be provided on both the anaesthesia module and ventilator module to confirm correct placement of the ventilator module when docking with the anaesthesia module.

An aspect of the present technology provides a method for providing concentrated waste volatile anaesthetic to a recycling capture system by first removing waste gas from a patient circuit in a medical device, pressurising the gas, removing water vapour and then absorbing the waste anaesthetic onto an absorbent material under pressure, with subsequent desorption of waste anaesthetic under vacuum at the end of the procedure and passage under controlled-flow conditions into a recycling canister.

Water vapour may, for example, be removed using a Nafion (RTM) membrane. Nafion is a sulfonated tetrafluoroethylene-based fluoropolymer-copolymer.

There is also disclosed (but not claimed) a method for providing concentrated waste volatile anaesthetic under ideal flow conditions to a recycling capture system by first removing waste gas from the patient circuit in a medical device, pressurising the gas, removing water vapour using a Nafion membrane and then absorbing the waste anaesthetic onto an absorbent material under pressure, with subsequent desorption of waste anaesthetic under vacuum at the end of the procedure and passage under controlled-flow conditions into the recycling canister.

In some embodiments the pressure in a pressurised absorption canister is between 1 and 4 bar. In some embodiments the pressure in a pressurized absorption canister is between 4 and 20 bar.

In some embodiments the pressure in a pressurized absorption canister is between 0 and 1bar.

A further aspect of the present technology provides a method for the dehumidification of waste anaesthetic gases containing volatile anaesthetic agent by the passage through Nafion tubing, with or without prior pressurization, for temporary capture and storage during anaesthesia with subsequent discharge of dehydrated waste gases to a volatile anaesthetic recycling capture system either during anaesthesia or after the conclusion of the episode of anaesthesia.

A further aspect of the present technology provides the dual use of a pump or turbine for the pressurization of waste anaesthetic gases containing volatile anaesthetic for capture of volatile anaesthetic agent and also for the pressurization of air to supply compressed air to provide to the patient by the anaesthesia machine.

Some aspects and embodiments may provide for the passage of waste gases without volatile anaesthetic during intravenous anaesthesia to atmosphere rather than to a volatile anaesthetic recycling system.

Some embodiments provide for the use of a diaphragm pump for the pressurization of waste gases and compressed air.

Some embodiments use a turbine for the pressurization of waste gases and compressed air.

In some embodiments a diaphragm pump pressurises waste gases for anaesthetic recovery and also provides compressed air for ventilator

Some embodiments involve Nafion-based moisture removal (for example using PEEP waste O2 as a drying gas).

In some embodiments no pipeline air required; instead this may be supplied by a turbine in ventilator mode and by a diaphragm pump in anaesthesia mode

A turbine may be provided for ventilation and recirculation. Air may be drawn in from atmosphere during ventilator mode and from recirculation during anaesthesia (with pressurised fresh air now supplied from diaphragm pump).

### Waste air/oxygen may pass to atmosphere during TIVA

The volatile anaesthetics may be one or more of: Sevoflurane; Desflurane; and Isoflurane.

The absorbent material in a pressurised canister may be silica gel, activated carbon, aerogel (silica or carbon based), metal organic framework, halocarbon-modified silica gel or carbon or plain carbon or graphite.

Where waste gases from the medical device do not contain volatile anaesthetic they may be passed directly to atmosphere without passing through the pressurised absorbent.

The pressurisation system may be able to take atmospheric air in and pressurise it to supply the medical device with pressurised air.

The pressurised air may be able to feed an oxygen concentrator and provide oxygen to the medical device.

In some embodiments the technology is modular. The anaesthesia module can be separated from the ventilation module. This means that the machine can stay with the patient through theatre and then into intensive care, with the anaesthesia module being returned to theatre for the next case. This means the same user interface and controls are used in both environments.

The technology separates the delivery of ventilation from the maintenance of circuit volume and waste. The waste gas stream is pressurised by a diaphragm pump and dehumidified by passage through a Nafion membrane bundle before the waste anaesthetic is absorbed under pressure onto an absorbent in a pressure-tolerant container. The Nafion is supplied with dry oxygen from the PEEP valve waste stream to remove water from the pressurised waste gas. Waste oxygen/air passes out of the vessel through a proportional solenoid valve controlled by the anaesthesia machine.

In an aspect of the technology, dry waste can be desorbed under vacuum when the pump is operated in reverse and re-used in the same patient or is passed into a separate, removable, recycling absorbent at the end of the operation under optimal flow conditions. In another aspect, waste anaesthetic is simply passed into an exhaust canister by depressurising the canister through the proportional flow valve with direction into the canister under control of a three-way valve.

Separate waste/ventilation systems allow:
- Use of Nafion water removal systems that do not need regeneration but would create too much backpressure in normal circuits
- The re-delivery of gases wasted at the start of anaesthesia during the maintenance phase
- Much smaller, higher efficiency recycling absorbent systems, working at lower flows
- No flow through the absorbent during cases not using volatile anaesthetic, reducing anaesthetic losses
- No scavenging flow through the recycling absorbent, reducing anaesthetic loss and contamination
- Use of absorbent in the machine (44-V1) with lower binding energies so that bound anaesthetic may be removed more easily at lower pressures/vacuum (e.g. Silica gel or macroporous activated carbon over microporous activated carbon).
- Use of absorbent in the recycling canisters (44-V2) with lower binding energies so that the captured anaesthetic may be removed for recycling more easily allowing multiple cycling of absorbent material.

Control systems in the anaesthesia machine may be separated into those required for ventilation/anaesthesia function and those for data analysis. Data may be passed between the modules by CANBUS, UART, SPI, I2C, Modbus, ASCII or other machine communication schema.

The data analysis module may, for example, comprise either a Field Programmable Gate Array, a Graphics Processor Unit (GPU)-based Artificial Intelligence (AI) system, for example a Tensor Processing Unit (TPU), or a heterogeneous multicore processor based (with or without a GPU) on a System on Module (SoM) although other systems can be used familiar to those skilled in the art. In one embodiment, the Al module is detachable from the ventilator module and contains patient-specific analysis and data.

In another embodiment, Al or Machine Learning (ML) is provided by the visualisation controller within the ventilator module.

In one embodiment, Al-based analysis may be displayed on a different display device attached to the unit. In another embodiment, Al-based analysis may be displayed within other data in the primary and secondary display. Al-based analysis may refer to:
- Machine learning
- Automation
- Analysis of ventilator performance and Al-driven ventilator parameter adjustment suggestions
- Analysis of vital signs with suggestions on therapy
- Derivation of risk to inform skilled clinicians of a 'need to rescue'
- Analysis of the impact of pharmaceutical management in relation to the above
- Speech recognition for hospital staff so that simple commands such as "[model] [unit number], please read out current vital signs from [patient]."

Other algorithms can be added and used under regulatory control as required.

In this way, edge AI can add to the performance of the machine without compromising the performance of the core functionality of the device.

In the case of a module that can be disconnected from the ventilator module, as this analysis is specific to the patient during their stay, the device can stay with the patient when they are nolonger connected to the anaesthesia machine/ventilator, but are connected to a piece of compatible monitoring hardware. The Al device is detached from the ventilator module and inserted into such a compatible monitoring system. In the same way, the Al device may be recording data from a patient under monitoring following their admission. When critical care intervention is required, the Al device can be removed from the ward-based monitor and inserted into the ventilator module, uploading the patient details, history and episode-related data which can then be added to during their stay in critical care.

The present technology provides a system for the unbroken acquisition of data from the entire patient stay in critical care, emergency areas and theatres in a single device by including a visualisation controller or Al module within a portable ventilator module that is capable of storing long-term data, and performing artificial intelligence, machine learning analysis and automation and wherein the portable ventilator module can provide life-sustaining ventilation support to a patient in critical care and wherein the portable ventilator can dock with an anaesthesia module to provide anaesthesia during operations in theatre so that a machine change from an ICU ventilator to a separate anaesthesia machine and back again is not required.

The present technology also provides a system for the acquisition of data from the entire patient stay in an Artificial Intelligence module that is capable of performing analysis of the data wherein this module can be inserted into a portable ventilator module to provide life-sustaining ventilation support to a patient in critical care and wherein the portable ventilator can dock with an anaesthesia module to provide anaesthesia during operations in theatre.

The Al module may include hardware dedicated to the performance of machine learning or artificial intelligence such as field-programmable gate arrays, graphical processor units or dedicated Al units.

The visualisation module may include hardware dedicated to the performance of machine learning or artificial intelligence such as field-programmable gate arrays, graphical processor units or dedicated Al units.

The Al module may provide storage facility for patient information and data generated while connected to a compatible device.

The visualisation module may provide storage facility for patient information and data generated while connected to a compatible device.

The Al module may include a Heterogeneous Multicore Processor on a System on Module to provide high and low-power processors for the provision of different algorithms along with data storage, power management, camera and microphone access.

The visualisation module may include a Heterogeneous Multicore Processor on a System on Module to provide high and low-power processors for the provision of different algorithms along with Graphical User Interface (GUI) software, data storage, networking, power management, camera and microphone access.

The Heterogeneous Multicore Processor on a System on Module may provide the ability to support high resolution displays with associated graphical user interface software to display Algenerated information on a separate display or an integrated display.

When the patient stay has ended, the patient-specific data is deleted. Alternatively the patient-specific data could be stored in a secure, regulatory compliant archive. In both scenarios, the Al device can be used for another patient

In a further embodiment, the visualisation module can be connected to the internet via a gateway device for security.

In another embodiment the visualisation module may be connected directly to the internet and use a secure operating system with or without additional cybersecurity.

The clinical user may ask the ventilator module for help. The machine may then request help from a central system in the cloud. The system of some embodiments is able to notify other ventilator modules or Al modules connected to the cloud network that help is required. Certain trained and accredited clinical users are then able to respond to the request by offering clinical advice to the clinical user in need of assistance. The remote user would be able to see the readings and analysis from the machine requiring help on their user interface and communicate directly by audio or visual means. In this way, help can be provided remotely.

In another embodiment, this system can be set up outside of the internet and use only the hospital intranet, so that help can be offered by designated staff to the wider hospital users if required and patient data can be checked to ensure appropriate identification to the specialist required.

The present technology provides a system whereby the user may use a portable ventilator module with a connection to the internet or hospital intranet to request help from other experienced clinicians wherein the portable ventilator module may transmit data including audio and/or visual information to another compatible device used by an experienced remote user, and in which the compatible device may be the same or similar to the portable ventilator module, with transmission of data, audio and/or visual information from the remote portable ventilator module to the original portable ventilator module to aid the user in need of assistance. An embodiment wherein the request for help may be seen across the network of portable ventilator modules and in which an available experienced remote user with correct access privilege may accept the request for help.

An embodiment wherein the portable ventilator module is capable of docking with an anesthesia module to provide anaesthesia to patients undergoing surgery and the request for help can be accepted by any available experienced clinicians in the theatre complex.

In an embodiment, Mass Flow Controllers are used to provide air and oxygen and drive ventilation pressure. The devices can respond to changes in flow within 1-5ms. Therefore, the flow profile can be controlled within the breath very accurately. By analysing the flow and pressure trace during the breath, the mass flow controllers are able to adjust the flow rate to deliver the required volume within the same breath. This is not possible on other pressure modes, where the volume must be adjusted for subsequent breaths. Furthermore, this process is done with the PEEP valve fully closed, so that no flow-by is allowed. This means that all of the gas goes into the patient and doesn't bypass the patient. This is much more efficient for oxygen consumption. Finally, at lower frequencies, the breath flow-profile can be shaped to fit the flow profile as the patient passive exhales. This means that if different sections of the lungs have different time constants (as demonstrated on passive expiration), this difference is accounted for and different lung units are not exposed to over-pressure or volume. This is a different form of ventilation than that currently offered and may enable ventilation at lower pressures, volumes and frequencies, reducing dynamic hyperinflation and ventilator-induced lung injury. In another embodiment of the invention, this flow-profile is analysed by the Al module in conjunction with the capnography (carbon dioxide) and pulse oximetry or arterial gas analysis to deliver the lowest volumes and pressures required for adequate arterial oxygenation and carbon dioxide removal.

Different aspects and embodiments of the technology may be used separately or together.

Further particular and preferred aspects of the present invention are set out in the accompanying independent and dependent claims. Features of the dependent claims may be combined with the features of the independent claim as appropriate, and in combination other than those explicitly set out in the claims. Each aspect can be carried out independently of the other aspects or in combination with one or more of the other aspects.

Embodiments of the present technology are shown, by way of example, in the accompanying drawings.

Example embodiments are shown and described in sufficient detail to enable those of ordinary skill in the art to embody and implement the systems and processes herein described. It is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate. The invention is not limited in the design and shape of the structure shown in the drawings.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Detailed Description of Drawings:

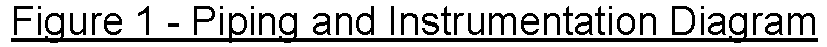

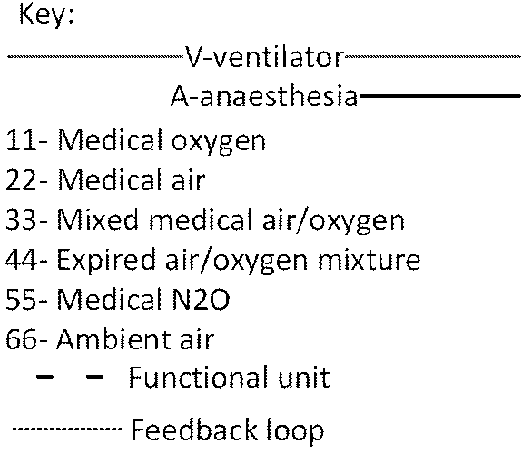

A separate anaesthesia module detaches from ventilator module.

When the ventilator module is operating independently. Pressurised oxygen from tank 11-CY1 passes through a filter 11-F2 and one way valve 11-CV2 to a pressure reducing valve 11-PRV1, reducing the pressure to 3 bar. The pressure in the oxygen cylinder 11-CY1 is monitored by 11-PT2. The pressure downstream of 11-PRV1 is monitored by 11-PT1. 11-PT1 also detects the pipeline oxygen pressure from ISO-standard connectors 11-C1. Pipeline oxygen passes through the filter 11-F1 and one way valve 11-CV1 to prevent reverse flow. Pipeline oxygen is supplied at 4-6 bar and therefore, oxygen supplies from 11-C2 should only be used if the pipeline pressure drops below 3 bar. 11-PRV2 reduces the pressure to 2.6 bar, monitored by 11-PT3. The flow of oxygen into the patient circuit is controlled by 11-PFV1 with measurement from 11-FM1. When in ventilator-only mode, oxygen is mixed with ambient air drawn from the air inlet 44-C3 and monitored by oxygen sensor 33-O2T1 and temperature sensor 33-TT1. Air and oxygen are drawn into the dynamically controlled turbine 33-T1, which pressurises the air and oxygen to drive inspiration and maintains an appropriate speed to prevent back-flow of gas during the remainder of the respiratory cycle. The inspiratory pressure is monitored by 33-PT1 and the flow monitored by 33-FT1 with outlet temperature monitored by 33-TT2. Inspiratory gases pass through the ISO standard connector 33-C1 into the disposable patient circuit tubing external to the machine. A near-patient flowmeter 44-FT1 and pressure transducer 44-PT1 are connected between the y-piece and the patient connection (e.g. Endotracheal tube). The near patient devices are connected to the device for power (3.3-5v) and communication (e.g. UART). Inspiratory and expiratory gases pass to and from the patient via a Heat and Moisture Exchange (HME) filter 44-F1. Pressure relief can be placed in the inspiratory or expiratory limbs or near-patient as shown. Expiratory gases return to the device via the standard ISO connector 44-C1 with flow monitoring by 44-FT2. A water trap 44-WT1 removes any liquid water from the circuit.

The pressure of the patient circuit is controlled by a Positive End Expiratory Pressure (PEEP) valve 44-PEEPV1. The PEEP valve control can be but is not limited to a diaphragm-type with gas pressure from the oxygen supply regulated by inlet and outlet proportional valves under the control of a pressure transducer and microcontroller. The PEEP valve may also be but is not limited to control by voice coil. Exhaust gases exit through the expiratory connector 44-C2 to atmosphere. Further features are offered including pressurised oxygen for a nebuliser and cuffpressure control system.

When the anaesthesia module is connected to the ventilator module. In one embodiment, the oxygen delivery system described above (reference to V-Gas conditioning) is used to provide oxygen into the device. In another embodiment of the invention, an additional supply of pressurised oxygen can be provided as explained following. Using a separate supply of oxygen may be preferred as connections to different pipeline or cylinders are not required to be made/unmade and because the flows through flow control valves (e.g. 11-PRV1) are different for dynamically mixing systems required for the ventilator only function (up to 200SLPM) whereas flows of 0-20SLPM are required for closed-circuit anaesthesia, with greater accuracy at low flows of 150-500ml/min.

Pressurised oxygen, air and nitrous oxide are delivered via identical pressure control and monitoring systems as described for "V-Gas Conditioning" to deliver filtered, pressurised gas at 2.6bar to a flow control valve. In this case, a piezo-electric flow controller is used to deliver gas into the patient circuit at the rate demanded by the control system, although other methods familiar to those experienced in the state of the art can be used. A bypass oxygen supply is provided via manual flowmeter 11-FM2 and safety isolation valve 11-SV1.

Volatile anaesthetic can either be added by the incorporation of a vapouriser or by the use of a volatile injection system downstream of the multi-gas outlet. Gas is drawn through the one way valve 44-CV2 and connection between the anaesthesia and ventilator modules 44-C3 into the ventilator module for delivery to the patient. Exhaled gases return through the expiratory port 44-C2 to the CO₂ absorber 44-CO2A and volume reservoir bag 44-BAG to complete the closed circle circuit. In this way, exhaled gases return via the PEEP valve to the bag. These gases are then drawn into the turbine and delivered into the inspiratory limb following additions of further air/oxygen/nitrous oxide/volatile anaesthetic as demanded by the anaesthesia controller. Any excess circuit volume is wasted through the Adjustable Pressure Limting valve (APL- A-APL). This valve has a minimum value of 2cmH₂O to ensure the circuit remains filled. When the turbine is in operation, the APL valve is set to minimum. If the user wants to manually ventilate the patient, the APL valve is turned to the desired pressure value by the user. This switches off the turbine and will pressurise the circuit as demanded by the user via the APL valve. Exhaust gases pass to a two position, two-way valve 44-EV1 that directs waste gas to the active scavenging system via 44-C5 or a passive, recycling scavenging system via 44-C4. Positive pressure relief valve 44-PRV2 protect the patient circuit from overpressure in the case of occlusion and a negative pressure relief valve 44-PRV2 protects the patient circuit from excess negative pressure from the active scavenging system. Condensation of gases with moisture from the patient, humidifier or CO₂ absorber is prevented by the heating of the anaesthesia manifold by Man-Htg1 under the control and monitoring of temperature transducers Man-T1/T2. Patient circuit humidity and temperature are monitored by 44-H1 and pressure in the anaesthesia manifold (pre-turbine) by 44-PT2.

### Figure 2

The ventilator component on the ventilator processor interfaces with the I/O to manage the ventilator module. The visualisation controller provides an interface to the network, the Primary UI and also to the Secondary UI, but in the latter case, this is provided through the docking port that interfaces between the ventilator and anaesthesia modules. The Anaesthesia Processor interfaces with the I/O in the anaesthesia module and also the Anaesthesia Module UI to display important parameters specific to the Anaesthesia module. Communication between the processors is via CANBUS. The CANBUS connection is passed via the docking port between the anaesthesia and ventilator modules to allow the modules to work together. Power in the anaetshesia module is provided from a separate power controller (not shown) in the anaesthesia module. This provides power to the power controller in the ventilator module through the docking station (not shown). Likewise, a networking connection is passed from the anaesthesia module to the ventilator module to charge the portable ventilator module batteries when the ventilator module is docked to the anaesthesia module.

### Figure 3

The ventilator module 1 is docked to the anaesthesia module. The anaesthesia module includes a container for Carbon dioxide absorber 3, a reservoir bag 2, a secondary display 4, height adjust capabilities for the anaesthesia docking port, secondary display and table work area 5, a pole to attach intravenous pump racks 6, warming devices 7 or other ancillaries. The base 9 houses batteries and power connections, rails at the side 8 protect the device envelope and enable easy movement. The ventilator module houses the standard ventilator breathing system inspiratory and expiratory connections. These are in recessed housings to prevent damage (they are male connections) in the location shown 10 (actual connections not shown in model- only location).

### Figure 4

The ventilator module 21 is undocked from the anaesthesia module 22. The ventilator module is portable, with carrying handles 23 and the primary touchscreen user interface 24. A positioning recess 26 in the anaesthesia module ensures stability when the ventilator module docks to the anaesthesia module docking port 27. Volatile anaesthesia may be provided by a volatile anaesthetic-specific removable cassette 25 located within the anaestheia module. The anaesthesia module also contains backup gas cylinders 28 and connections to pipeline gas supplies (not shown).

### Figure 5

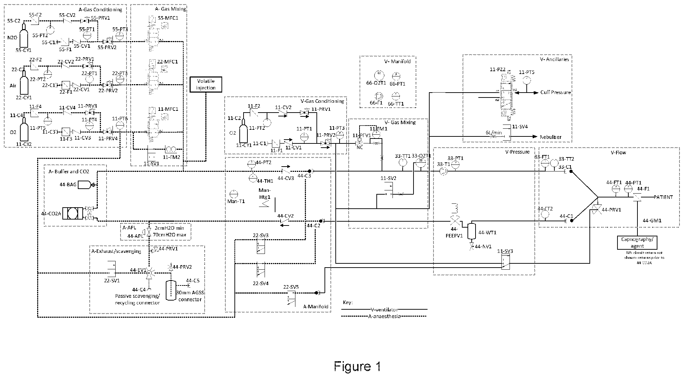

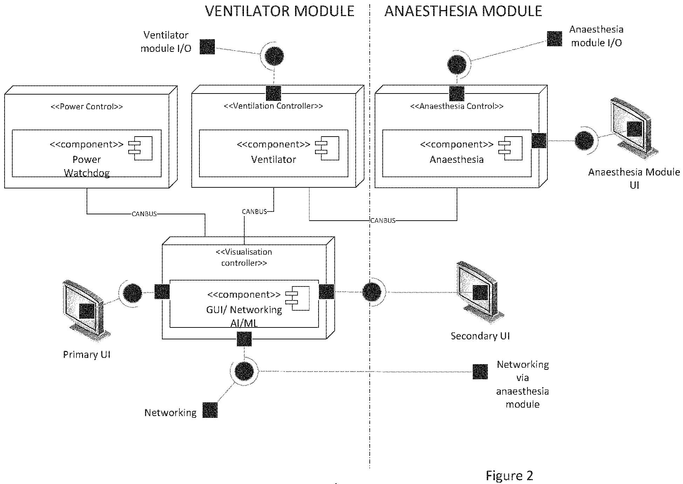

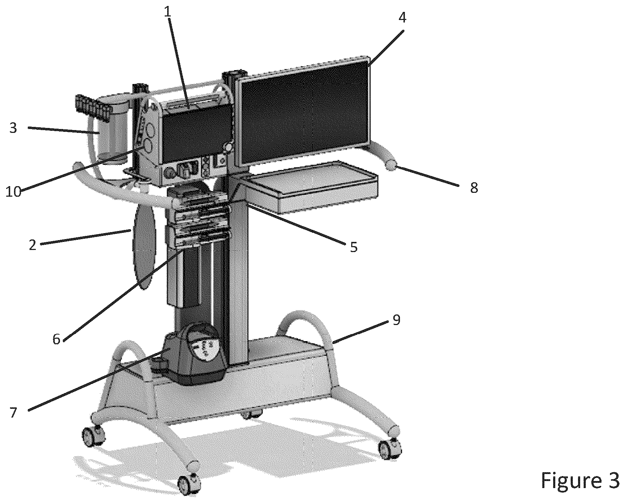

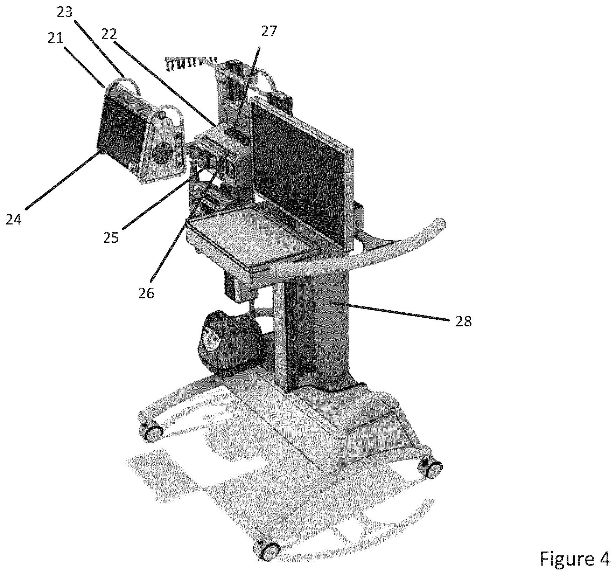

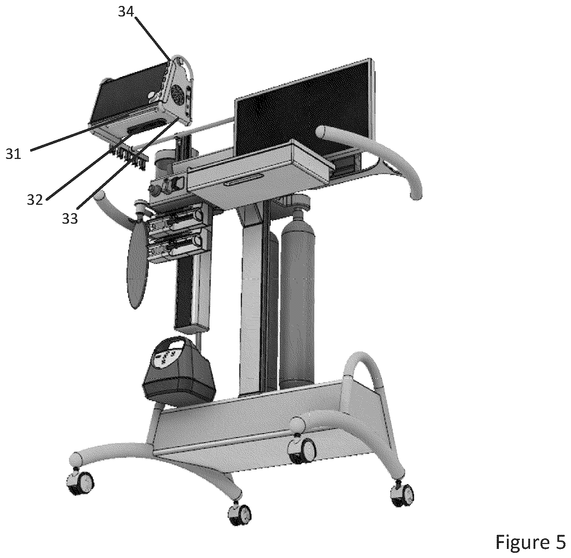

The ventilator module location strip 31 matches to the anaesthesia module location recess (see figure 4). The male docking port 32 of the ventilator module is protected by the protrusion of the support frame 33. Other exposed buttons and rotary encoder are contained on an easily removable section 34 so that they can be replaced quickly if damaged and will not damage the main unit.

### Figure 6

A single ventilator module with networking, data storage and Artificial Intelligence (Al)/Machine Learning (ML) capability is used across critical care areas to provide uninterrupted data capture and analysis. Additional functionality is provided by a docking port that enables extra display of information in Intensive Care Units (ICU), Emergency Department (ED), Wards, Radiology and Recovery and docking with an anesethesia module allows the provision of volatile anaesthesia for surgery in theatres with the potential to integrate with volatile anaesthetic recycling services to reduce necessary infrastructure and reduce environmental pollution. At all stages, transfer is with a fully-functional ICU-grade ventilator and a high frequency data record is maintained with Al/ML-based analysis.

AI/ML in devices and their connection to the network enables the passage of data and analysis across the network to modules in critical care so that patients at risk of deterioration can be identified to experienced clinciians, their data and analysis reviewed and acted upon. Data transfer and analysis also allows review of issues in one theatre by another and the review of patients in recovery by a device in theatre (for example, a patient waking up would be reviewed by the anaesthetist in theatre).

### Figure 7

An Al module is inserted into a compatible ward-based monitor. The monitor and Al module are then used to monitor a patient on the ward. The Al module stores patient information and performs analysis including the ability to alert expert clinicians to the 'need to rescue'. This refers to the need to review a deteriorating patient. When the patient needs critical care intervention, the Al module is removed and inserted into the ventilator module, bringing with it patient information, history and data. This ventilator module is used in ICU and can also dock with an anaesthesia module to provide anaesthesia in theatre. It is then returned to the ward-based monitor when the episode of critical care is complete.

### Figure 8

In ventilator only mode. Oxygen from a cylinder or pipeline supply, is supplied through a filter 11-F1 with monitoring by pressure transducer 11-PT1. The flow control valve 11-PFV1 regulates flow into the patient circuit through the mass flow meter (11-PT2 atmospheric pressure, 11-TT1 temperature transducer, 11-FT1 flowmeter). Ambient air is drawn into circuit through the 3 port 2 position valve 11-3P2PV1 and filter 22-F1 under monitoring from 22-PT1. Flow control valve 22-PTV1 and the mass flow meter (22-PT2, 22-TT1, 22FT1) regulate flow generated by the turbine 33-T1 under the control of inspiratory flowmeter 33-FT1 and near-patient differential pressure transducer 44-DPT1. Oxygen concentration is monitored by 33-OT1. Electronically-controlled Patient pressure relief and power-off gas supply is provided by 33-NOSV1, with a mechanical relief valve 33-PRV1. Air is filtered by 33-F1. A standard ISO connector connects to the ventilator breathing system. Near-patient flowmeter is provided prior to the near-patient differential pressure transducer. Exhaled gas returns to the ISO-standard connection port, exhaled and bypass flow is measured by the flowmeter 44-FT1 with circuit pressure controlled by the PEEP valve 33-PEEPV1. This may be controlled by air loaded diaphragm (may be from a dual valve pressure regulator 33-EPCD) or by voice coil. In ventilator-only mode, exhaust gas leaves from the exhaust port following the PEEP valve 44-PEEPV1.

When the anaesthesia module is connected, the circuit is closed. Exhaust gases from the PEEP valve 44-PEEPV1 pass to the CO2 absorber 44-CO2ABS1 and to the reservoir bag 44-BAG1. Recirculated gases pass through the normally-closed valve 33-NCSV1 to return to the turbine 33-T1. Ambient air is no longer drawn into the patient circuit by the turbine 33-T1 as the 3 port 2-position valve 11-3P2PV1 is directed instead to compressed air stored in 11-V1. Compressed air is provided by the air compressor 44-DP1 to a pressure of 4-6 bar. Preferably 44-DP1 is a diaphragm pump, although other pumps familiar to those skilled in the art can be used. As an alternative embodiment, a turbine may be used and the pressure in 11-V1 be reduced to 20-100mbar. Ambient air can be drawn to into the inlet of the pump/turbine from atmosphere or from exhaust gases. Exhaust gases are passed from the patient circuit to 44-DP1 by the action of 44-3P2PV1 when the controller detects excess pressure in the circuit following the PEEP valve (44-CO2ABS1, 44-BAG1). Exhaust gases are filtered first by 44-F1. During exhaust, gases pass through 44-3P2PV1 to the pump 44-DP1 through the three-port valve 44-3P2PV2 to Nafion water-selective tubing 44-MF1 to remove water vapour from exhaust gases. Dried gas passes to the capture chamber 44-V1 at pressure of atmosphere to 4 bar controlled by the flow control valve 44-PFV1 with scrubbed waste gases passing to atmosphere. The capture chamber contains an absorbent material such as silica, activated carbon, aerogel (silica or carbon-based), graphene, zeolite or metal-organic framework (MOF). The volatile anaesthetic is captured onto the capture material at pressure. At the end of the operation, when the anaesthesia machine is no longer being used, the capture chamber 44-V1 is purged of volatile anaesthetic first by passing through 44-MF1, 44-3P2PV2, 44-3P2PV1 and being directed to the removable capture canister 44-V2, in which dried volatile anaesthetic is absorbed with scrubbed waste gases passing to atmosphere. A vacuum can be provided by pump 44-DP1 operating in reverse to complete volatile anaestehtic desorption from 44-V1 and transfer to 44-V2. The storage canister 44-V2 can be removed for further recycling according to other prior art (for example WO2016/027097 and WO2017/144879). By this method, the pump 44-DP1 can perform three functions at different stages of the anaesthesia case:
1. Air compression to provide compressed air to be added to the patient circuit (mostly before case starts and the machine is turned on or rarely and intermittently when the exhaust is not active)
2. Dehumidification of exhaust gases and storage of volatile anaesthetic in chamber 44-V1 (when 44-BAG1 pressure high during the operation)
3. Transfer of stored volatile anaesthetic from 44-V1 to a removable anaesthetic recycling canister 44-V2 at the end of the operation or day (depending on the filling status of the absorbent canister).

### Figure 9

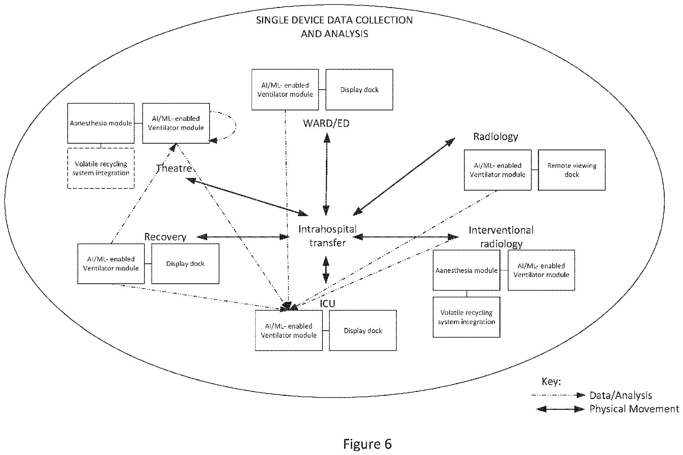

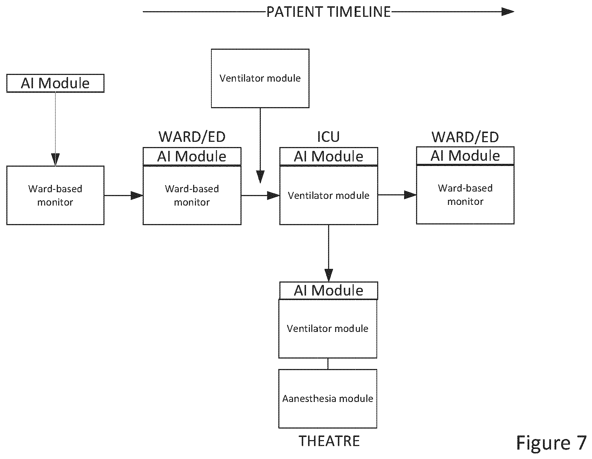

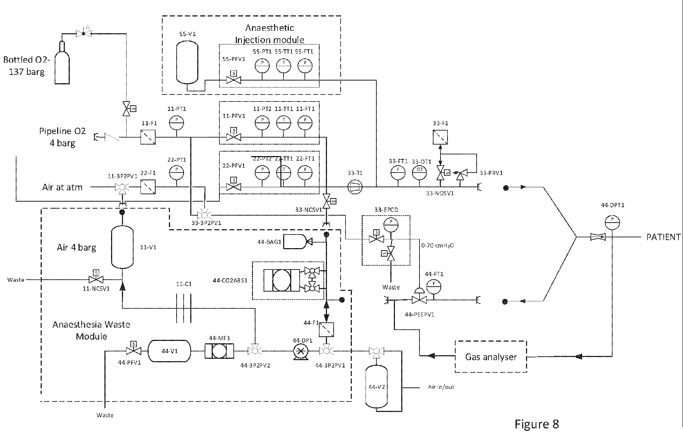

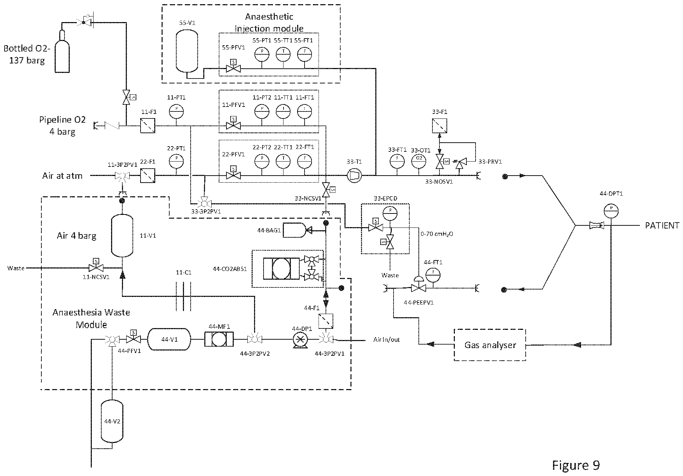

The patient circuit is arranged in the same way as figure 6, to provide modular anaesthesia and ventilation so that the device may perform as a stand-alone portable ventilator using ambient air and pressurised oxygen and an anaesthesia machine where the patient circuit is closed and air and oxygen are both provided from a pressurised source. The difference between figure 6 and 7 is the configuration of the waste processing and recycling system and the provision of pressurised air in the anaesthesia module

Exhaled gases pass through the PEEP valve 44-PEPV1 in the ventilator module into the anaesthesia module. Excess pressure in the circuit leads to wasting of exhaust gases through filter 44-F1 and valve 44-3P2PV1 to the pump or turbine 44-DP1. This pressurises the exhaust gases to 4 bar through the Nafion membrane 44-MF1 to remove moisture and then to the collection chamber 44-V1 where absorbent material as described for Figure 6 is located. Nafion moisture removal (uses PEEP waste O2 as drying gas). The absorbent material scrubs the dried exhaust gases of volatile anaesthetic. Scrubbed waste gas above 4 bar spills over the proportional valve 44-PFV1 and is subsequently directed to atmosphere as waste. Waste air/oxygen stripped of anaesthetic from exhaust passes into theatre air. Following the completion of the case, the proportional valve 44-PFV1 opens to gradually transfer the contents of the collection chamber 44-V1 to the removable absorbent canister 44-V2, as used by recycling systems for example (for example WO2016/027097 and WO2017/144879). Recycling canister (44-V2) collects evacuated contents off 44-V1 at end of case. Before the start of the case or if the air pressure is low in the pressurised air reservoir 11-V1, the pump 44-DP1 compresses air taken from atmosphere through 44-3P2PV1 and passes it to 44-3P2PV2 through the connection 11-C1 and to the air reservoir 11-V1 at 4 bar. If required, 11-V1 can be vented to atmosphere by passing its contents through 11-NCSV1.

Compressed air may be provided during anaesthesia mode.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

Although illustrative embodiments have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims.

## Claims

1. A modular system for the unbroken acquisition of data from an entire patient stay in critical care, emergency areas and theatres, the system including a portable ventilator module (21) in combination with an anaesthesia module (22), the portable ventilator module is detachably attachable to the anaesthesia module, wherein the portable ventilator module can provide life-sustaining ventilation support to a patient in critical care and wherein the portable ventilator module can dock with the anaesthesia module to provide anaesthesia during operations in theatre so that a machine change from an intensive care unit (ICU) ventilator to a separate anaesthesia machine and back again is not required, thereby avoiding data loss, **characterised in that** the portable ventilator module comprises a turbine and, when the anaesthesia module is not connected, air is drawn in from atmosphere by the action of the ventilator module turbine, and wherein a docking port (27) is provided to connect the ventilator module to the anaesthesia module via a single location, in which a connection is provided within the docking port to connect recirculated gas from the anaesthesia module to the air intake input in the ventilator module.

2. A system as claimed in claim 1, comprising an Artificial Intelligence (AI) module which can be inserted into or form part of the portable ventilator module.

3. A system as claimed in claim 2, wherein the Al module is detachable from the portable ventilator module and contains patient-specific analysis and data.

4. A system as claimed in claim 2 or claim 3, in which when critical care intervention is required, the Al module can be removed from a ward-based monitor and inserted into the ventilator module, uploading the patient details, history and episode-related data which can then be added to during their stay in critical care.

5. A system as claimed in any preceding claim, wherein ambient air is mixed with oxygen for ventilation when the ventilation module is not connected and wherein pressurised air is mixed with pressurised oxygen when the anaesthesia module is connected.

6. A system as claimed in any preceding claim, comprising an anaesthesia module controller that interfaces with components in the anaesthesia module and communicates with the ventilator module.

7. A system as claimed in any preceding claim and further comprising a display for the anaesthesia module, independent of the ventilation module and active when the anaesthesia module is powered on but not necessarily connected to the ventilation module, for parameters specific to the anaesthesia module.

8. A system as claimed in any preceding claim, in which the ventilation module provides an interface for a secondary display via a docking port that can also be used to connect to the anaesthesia module.

9. A system as claimed in any preceding claim, in which a connection is provided within the docking port to connect the exhaled gas from the patient from the ventilator module with the recirculation intake of the anaesthesia module.

10. A system as claimed in any preceding claim, in which an ethernet connection is provided within the docking port to link the ethernet output from the ventilation module to a healthcare institution Local Area Network.

11. A system as claimed in any preceding claim, in which the ventilator module can operate independently of the anaesthesia module.

12. A system as claimed in claim 2, in which the Al module includes hardware dedicated to the performance of machine learning or artificial intelligence such as field-programmable gate arrays, graphical processor units or dedicated AI units.

13. A system as claimed in any preceding claim, whereby the portable ventilator module is configured to be connected to the internet or hospital intranet such that a user may use the portable ventilator module to request help from other experienced clinicians wherein the portable ventilator module is configured to transmit data including audio and/or visual information to another compatible device used by an experienced remote user, wherein the compatible device is the same or similar to the portable ventilator module, with transmission of data, audio and/or visual information from the remote portable ventilator module to the original portable ventilator module to aid the user in need of assistance.

14. A system as claimed in claim 13, wherein the request for help may be seen across a network of portable ventilator modules such that an available experienced remote user with correct access privilege may accept the request for help.

15. A system as claimed in claim 14 when depending on claim 2 or claim 3, wherein the AI module is configured for establishing connection to the network to enable the passage of data and analysis across the network to modules in critical care so that patients at risk of deterioration can be identified to experienced clinicians, their data and analysis reviewed and acted upon.

## Patentansprüche

1. Modulares System zur lückenlosen Erfassung von Daten eines gesamten Patientenaufenthalts in der Intensivpflege, in Notfallstationen und in Operationssälen, wobei das System ein tragbares Beatmungsmodul (21) in Kombination mit einem Anästhesiemodul (22) umfasst, wobei das tragbare Beatmungsmodul abnehmbar am Anästhesiemodul angebracht werden kann, wobei das tragbare Beatmungsmodul lebenserhaltende Beatmungsunterstützung für einen Patienten in der Intensivpflege bereitstellen kann und wobei das tragbare Beatmungsmodul an das Anästhesiemodul anschliessbar ist, um während Operationen im Operationssaal eine Anästhesie bereitzustellen, so dass ein Gerätewechsel von einem Beatmungsgerät einer Intensivstation (ICU) zu einem separaten Anästhesiegerät und wieder zurück nicht erforderlich ist, wodurch Datenverluste vermieden werden, **dadurch gekennzeichnet, dass** das tragbare Beatmungsgerät eine Turbine umfasst und, wenn das Anästhesiemodul nicht angeschlossen ist, Luft aus der Atmosphäre durch die Wirkung der Turbine des Beatmungsmoduls angesaugt wird, und wobei ein Anschlussport (27) vorgesehen ist, um das Beatmungsmodul mit dem Anästhesiemodul über eine einzige Stelle zu verbinden, wobei ein Anschluss innerhalb des Anschlussports vorgesehen ist, um umgewälztes Gas von dem Anästhesiemodul mit dem Lufteinlass in dem Beatmungsmodul zu verbinden.

2. System nach Anspruch 1, umfassend ein künstliches Intelligenz (AI) Modul, das in das tragbare Beatmungsmodul eingesetzt werden kann oder einen Teil davon bilden kann.

3. System nach Anspruch 2, wobei das AI-Modul von dem tragbaren Beatmungsmodul abnehmbar ist und patientenspezifische Analysen und Daten enthält.

4. System nach Anspruch 2 oder Anspruch 3, bei dem, wenn ein Eingriff auf der Intensivstation erforderlich ist, das AI-Modul aus einem Monitor auf der Krankenstation entfernt und in das Beatmungsmodul eingesetzt werden kann, wobei die Patientendaten, die Krankengeschichte und ereignisbezogene Daten hochgeladen werden, die dann während des Aufenthalts auf der Intensivstation ergänzt werden können.

5. System nach einem der vorhergehenden Ansprüche, wobei Umgebungsluft mit Sauerstoff zur Beatmung gemischt wird, wenn das Beatmungsmodul nicht angeschlossen ist, und wobei Druckluft mit Drucksauerstoff gemischt wird, wenn das Anästhesiemodul angeschlossen ist.

6. System nach einem der vorhergehenden Ansprüche, umfassend eine Steuerung für das Anästhesiemodul, die über eine Schnittstelle mit Komponenten im Anästhesiemodul verbunden ist und mit dem Beatmungsmodul kommuniziert.

7. System nach einem der vorhergehenden Ansprüche, das ferner eine vom Beatmungsmodul unabhängige Anzeige für das Anästhesiemodul umfasst, die aktiv ist, wenn das Anästhesiemodul eingeschaltet ist, aber nicht notwendigerweise mit dem Beatmungsmodul verbunden ist, und die für das Anästhesiemodul spezifischen Parameter anzeigt.

8. System nach einem der vorhergehenden Ansprüche, bei dem das Beatmungsmodul über einen Anschlussport, der auch zum Anschluss an das Anästhesiemodul verwendet werden kann, eine Schnittstelle für eine sekundäre Anzeige bereitstellt.

9. System nach einem der vorhergehenden Ansprüche, bei dem ein Anschluss im Anschlussport vorgesehen ist, um das vom Patienten ausgeatmete Gas des Beatmungsmoduls mit dem Umwälzungseinlass des Anästhesiemoduls zu verbinden.

10. System nach einem der vorhergehenden Ansprüche, bei dem im Anschlussport ein Ethernet-Anschluss vorgesehen ist, um den Ethernet-Ausgang des Beatmungsmoduls mit einem lokalen Netzwerk einer Gesundheitseinrichtung zu verbinden.

11. System nach einem der vorhergehenden Ansprüche, bei dem das Beatmungsmodul unabhängig vom Anästhesiemodul betrieben werden kann.

12. System nach Anspruch 2, bei dem das AI-Modul Hardware enthält, die für die Durchführung von maschinellem Lernen oder künstlicher Intelligenz bestimmt ist, wie z. B. feldprogrammierbare Gate-Arrays, Grafikprozessoren oder spezielle Al-Einheiten.

13. System nach einem der vorhergehenden Ansprüche, wobei das tragbare Beatmungsmodul so konfiguriert ist, dass es mit dem Internet oder dem Krankenhaus-Intranet verbunden werden kann, so dass ein Benutzer das tragbare Beatmungsmodul verwenden kann, um Hilfe von anderen erfahrenen medizinischen Fachkräften anzufordern, wobei das tragbare Beatmungsmodul so konfiguriert ist, dass es Daten, einschließlich Audio- und/oder Bildinformationen, an eine andere kompatible Vorrichtung überträgt, die von einem erfahrenen entfernten Benutzer verwendet wird, wobei die kompatible Vorrichtung die gleiche oder eine ähnliche Vorrichtung wie das tragbare Beatmungsmodul ist, wobei Daten, Audio- und/oder Bildinformationen von dem entfernten tragbaren Beatmungsmodul an das ursprüngliche tragbare Beatmungsmodul übertragen werden, um dem Benutzer zu helfen, der Unterstützung benötigt.

14. System nach Anspruch 13, wobei das Hilfeersuchen über ein Netz von tragbaren Beatmungsmodulen gesehen werden kann, so dass ein verfügbarer erfahrener entfernter Benutzer mit korrekten Zugriffsrechten das Hilfeersuchen annehmen kann.

15. System nach Anspruch 14, wenn es von Anspruch 2 oder Anspruch 3 abhängt, wobei das AI-Modul so konfiguriert ist, dass es einen Anschluss an das Netzwerk herstellt, um die Weiterleitung von Daten und Analysen über das Netzwerk an Module in der Intensivpflege zu ermöglichen, so dass Patienten mit einem Verschlechterungsrisiko erfahrenen medizinischen Fachkräften gekennzeichnet werden können und ihre Daten und Analysen überprüft und entsprechend gehandelt werden kann.

## Revendications

1. Système modulaire pour l'acquisition ininterrompue de données sur l'ensemble du séjour d'un patient en soins intensifs, dans les zones d'urgence et les salles d'opération, le système comprenant un module de ventilateur portable (21) en combinaison avec un module d'anesthésie (22), le module de ventilateur étant amovible et pouvant être fixé au module d'anesthésie, dans lequel le module de ventilateur portable peut fournir un soutien de ventilation de maintien de la vie à un patient en soins intensifs et dans lequel le module de ventilateur portable peut s'amarrer au module d'anesthésie pour fournir une anesthésie pendant les opérations en salle d'opération, de sorte qu'un changement de machine d'un ventilateur d'unité de soins intensifs (ICU) à une machine d'anesthésie séparée et vice versa n'est pas nécessaire, évitant ainsi la perte de données, **caractérisé en ce que** le module de ventilateur portable comprend une turbine et, lorsque le module d'anesthésie n'est pas connecté, l'air est aspiré de l'atmosphère par l'action de la turbine du module de ventilateur, et dans lequel un port d'accueil (27) est prévu pour connecter le module de ventilateur au module d'anesthésie via un seul emplacement, dans lequel une connexion est prévue à l'intérieur du port d'accueil pour connecter le gaz recirculé du module d'anesthésie à l'entrée d'admission d'air dans le module de ventilateur.

2. Système selon la revendication 1, comprenant un module d'intelligence artificielle (IA) qui peut être inséré dans le module de ventilateur portable ou en faire partie.

3. Système selon la revendication 2, dans lequel le module d'IA est détachable du module de ventilateur portable et contient des analyses et des données spécifiques au patient.

4. Système selon la revendication 2 ou la revendication 3, dans lequel, lorsqu'une intervention de soins intensifs est nécessaire, le module d'IA peut être retiré d'un moniteur basé dans le service et inséré dans le module de ventilateur, en téléchargeant les détails du patient, l'historique et les données liées à l'épisode qui peuvent ensuite être ajoutées pendant son séjour en soins intensifs.

5. Système selon l'une quelconque des revendications précédentes, dans lequel l'air ambiant est mélangé à de l'oxygène pour la ventilation lorsque le module de ventilateur n'est pas connecté et dans lequel l'air sous pression est mélangé à de l'oxygène sous pression lorsque le module d'anesthésie est connecté.

6. Système selon l'une quelconque des revendications précédentes, comprenant un contrôleur de module d'anesthésie qui s'interface avec les composants du module d'anesthésie et communique avec le module de ventilateur.

7. Système selon l'une quelconque des revendications précédentes et comprenant en outre un écran pour le module d'anesthésie, indépendant du module de ventilateur et actif lorsque le module d'anesthésie est sous tension mais pas nécessairement connecté au module de ventilateur, pour les paramètres spécifiques au module d'anesthésie.

8. Système selon l'une quelconque des revendications précédentes, dans lequel le module de ventilateur fournit une interface pour un affichage secondaire via un port d'accueil qui peut également être utilisé pour se connecter au module d'anesthésie.

9. Système selon l'une quelconque des revendications précédentes, dans lequel une connexion est prévue à l'intérieur du port d'accueil pour relier le gaz expiré du patient du module de ventilateur à l'admission de recirculation du module d'anesthésie.

10. Système selon l'une quelconque des revendications précédentes, dans lequel une connexion Ethernet est prévue dans le port d'accueil pour relier la sortie Ethernet du module de ventilateur à un réseau local d'un établissement de soins de santé.

11. Système selon l'une quelconque des revendications précédentes, dans lequel le module ventilateur peut fonctionner indépendamment du module d'anesthésie.

12. Système selon la revendication 2, dans lequel le module d'IA comprend du matériel dédié à la performance de l'apprentissage automatique ou de l'intelligence artificielle, tel que des réseaux de portes programmables sur le terrain, des unités de processeur graphique ou des unités d'IA dédiées.

13. Système selon l'une quelconque des revendications précédentes, dans lequel le module de ventilateur portable est configuré pour être connecté à Internet ou à l'intranet de l'hôpital de sorte qu'un utilisateur puisse utiliser le module de ventilateur portable pour demander de l'aide à d'autres cliniciens expérimentés, dans lequel le module de ventilateur portable est configuré pour transmettre des données, y compris des informations audio et/ou visuelles, à un autre dispositif compatible utilisé par un utilisateur distant expérimenté, dans lequel le dispositif compatible est identique ou similaire au module de ventilateur portable, avec transmission de données, d'informations audio et/ou visuelles du module de ventilateur portable distant au module de ventilateur portable d'origine pour aider l'utilisateur ayant besoin d'assistance.

14. Système selon la revendication 13, dans lequel la demande d'aide peut être vue à travers un réseau de modules de ventilateur portables de sorte qu'un utilisateur distant expérimenté disponible avec un privilège d'accès correct puisse accepter la demande d'aide.

15. Système selon la revendication 14 lorsqu'il dépend de la revendication 2 ou de la revendication 3, dans lequel le module d'IA est configuré pour établir une connexion au réseau afin de permettre le passage de données et d'analyses à travers le réseau vers des modules de soins intensifs, de sorte que les patients à risque de détérioration puissent être identifiés par des cliniciens expérimentés, leurs données et analyses examinées et traitées.
